# EUROPEAN PATENT APPLICATION

(11) **EP 1 321 033 A1**
(43) Date of publication of application: **25.06.2003**
(21) Application number: 02258435.3
(22) Date of filing: 06.12.2002
(51) Int. Cl.: A01K 67/027, C12N 9/12

(54) **Transgenic expression of glycogen synthase kinase 3 in muscle**

(30) Priority: 21.12.2001 US 343479 P
(71) Applicant: Pfizer Products Inc., Groton, Connecticut 06340 (US)
(72) Inventor: Garofalo, Robert Sebastian, Pfizer Global R&D, Groton, Connecticut 06340 (US); Orena, Stephen Joseph, Pfizer Global R&D, Groton, Connecticut 06340 (US); Schachter, Joel Barry, Pfizer Global R&D, Groton, Connecticut 06340 (US)
(74) Representative: England, Peter Michael

(57) **Abstract**

The invention features a transgenic non-human mammal expressing or capable of expressing a GSK-3 transgene comprising a GSK-3 coding sequence, wherein expression of said coding sequence in the mammal is driven by an operably linked regulatory sequence that directs inducible expression in muscle. Following the induction of transgene expression, the transgenic non-human mammal exhibits hyperinsulinemia, increased weight gain on a high fat diet, and/or decreased muscle glycogen content on a high fat, as compared to a control animal not expressing the GSK-3 transgene. The invention also features methods of identifying *in vivo* modulators of GSK-3 activity.

## Description

This application claims priority, under 35 U.S.C. § 119(e) from U.S. provisional application 60/343,479, which was filed 12/21/2001.

### Field of the Invention

The invention features transgenic non-human mammals comprising a glycogen synthase kinase-3 (GSK-3) coding sequence that is operably linked to a regulatory sequence that directs inducible expression in muscle. These mammals exhibit hyperinsulinemia, increased weight gain on a high fat diet, and decreased muscle glycogen content on a high fat diet. The invention also features methods of identifying agents that modify *in vivo* GSK-3 activity.

### Background

GSK-3 was initially described as a key enzyme involved in glycogen metabolism, but is now known to regulate diverse cellular functions, including protein synthesis as well as glycogen metabolism (Cohen and Frame, Nature Reviews 2: 769-76, 2001).

Glycogen synthesis is a major pathway for glucose disposal in skeletal muscle following insulin stimulation. Glycogen synthase is the rate-limiting enzyme for synthesis, and its activity is reduced in type II diabetes (Thorburn et al., J. Clin. Invest. 85: 522-29, 1990; Beck-Nielsen et al., Diabetes Care 15: 418-29, 1992; Bogardus et al., J. Clin. Invest. 73: 1185-90, 1984), despite normal or elevated levels of glycogen synthase polypeptide in diabetic skeletal muscle (Vestergaard et al., J. Clin. Invest 91: 2342-50, 1993; Lofman et al., Am. J. Physiol. 269: E27-E32, 1995; Nikoulina et al., Diabetes 49: 263-71, 2000).

Previous studies have indicated that glycogen synthase kinase (GSK) 3 activity plays a role in the reduced glycogen synthase activity and reduced glucose disposal in diabetic muscle (Eldar-Finkelman et al., Proc. Natl. Acad. Sci. USA 93: 10228-33, 1996; Nikoulina et al., *supra*). GSK-3 protein levels and total activities are elevated in type 2 diabetic skeletal muscle, independent of obesity, and are inversely correlated with both glycogen synthase activity and maximally-stimulated glucose disposal (Nikoulina et al., *supra).*

GSK-3 deactivates glycogen synthase by phosphorylating it at serine residue sites designated 3a, 3b, and 3c (Cohen and Frame, *supra).* Human GSK-3 exists in two isoforms with molecular weights of 51 kd (GSK-3α) and 47 kd (GSK-3β). In rat, the two isoforms share 85% homology (Woodgett, Cancer Biol. 5: 269-75, 1994).

### Summary of the Invention

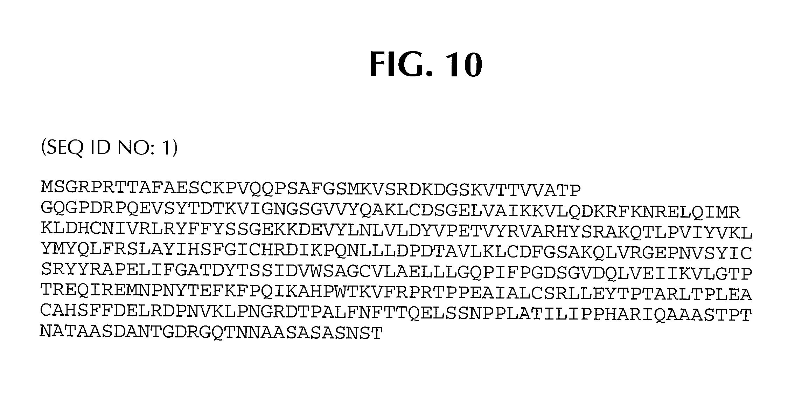

In the first aspect, the invention features a transgenic non-human mammal expressing or capable of expressing a GSK-3 transgene comprising a GSK-3 coding sequence, wherein expression of the coding sequence in the mammal is driven by an operably linked regulatory sequence that directs inducible expression in muscle, and wherein, following induction of transgene expression, the mammal exhibits hyperinsulinemia, hyperglycemia, increased weight gain on a high fat diet, and/or decreased muscle glycogen content on a high fat diet, as compared to a control mammal not expressing the GSK-3 transgene. Preferably, the inducible expression is muscle specific, more preferably, the regulatory sequence comprises a myosin light chain promoter. In other preferred embodiments, the transgenic mammal is a rodent, more preferably, a mouse, and the GSK-3 transgene is a human GSK-3α or GSK-3β coding sequence, more preferably, the human GSK-3β coding sequence of encodes the polypeptide of SEQ ID NO: 1, as shown in Fig. 10.

The second aspect of the invention features a method of identifying an agent that modulates GSK-3 activity *in vivo,* comprising contacting the agent with a transgenic non-human mammal expressing a transgene comprising a GSK-3 coding sequence, wherein expression of the coding sequence in the mammal is driven by an operably linked regulatory sequence that directs inducible expression in muscle, and wherein the non-human mammal exhibits hyperinsulinemia, hyperglycemia, increased weight gain on a high fat diet, and/or decreased muscle glycogen content on a high fat diet, as compared to a control animal not expressing the GSK-3 transgene, wherein a difference in insulin, glucose, body weight on a high fat diet, and/or muscle glycogen content on a high fat diet, in the presence of the agent and in the absence of the agent, is indicative that the agent modulates the activity.

In the third aspect, the invention features a muscle cell or myoblast isolated from a transgenic non-human mammal expressing or capable of expressing a GSK-3 transgene comprising a GSK-3 coding sequence, wherein expression of the coding sequence in the mammal is driven by an operably linked regulatory sequence that directs inducible expression in muscle, and wherein, following induction of transgene expression, the mammal exhibits hyperinsulinemia, hyperglycemia, increased weight gain on a high fat diet, and/or decreased muscle glycogen content on a high fat diet, as compared to a control mammal not expressing the GSK-3 transgene. In preferred embodiments, the muscle cell or myoblast is in primary culture or is immortalized.

Those skilled in the art will fully understand the terms used herein in the description and the appendant claims to describe the present invention. Nonetheless, unless otherwise provided herein, the following terms are as described immediately below.

By an "agent that modulates GSK activity" is meant a molecule which increases or decreases (including a complete elimination) the biological activity of a GSK-3 polypeptide.

An "agent that increases GSK-3 activity" refers to a molecule which intensifies or mimics the biological activity of a GSK-3 polypeptide. Such agents (i.e., agonists) may include proteins, nucleic acids, carbohydrates, small molecules, or any other compound or composition which increases the activity of a GSK-3 either by increasing the amount of GSK-3 present in a cell or by increasing the signaling of a GSK-3 polypeptide in its signal transduction pathway.

An "agent that decreases GSK-3 activity" refers to a molecule which inhibits or attenuates the biological activity of a GSK-3 polypeptide. Such agents (i.e., antagonists) may include proteins such as anti-GSK-3 antibodies, nucleic acids, carbohydrates, small molecules, or any other compound or composition which decreases the activity of a GSK-3 polypeptide either by reducing the amount of GSK-3 polypeptide present in a cell, or by decreasing the signaling of a GSK-3 polypeptide in its signal transduction pathway.

By "GSK-3 activity" is meant GSK-3-mediated phosphorylation of a GSK-3 substrate, e.g., glycogen synthase, eukaryotic initiation factor 2B (eIF2B), ATP citrate lyase, axin, β-catenin, adenomatous polyposis coli (APC), MUC1/DF3, cyclin D1, jun, myc, NFATc, C/EBPα, C/EBPβ, CREB, MITF,
HSF-1, tau, MAP1B, or presenilin 1.

By "glycogen synthase kinase (GSK) 3 coding sequence" is meant a vertebrate GSK-3 polynucleotide sequence encoding a GSK-3 polypeptide, including either the GSK-3α or GSK-3β isoform. Exemplary sequences are found in Genbank XM 029918 (human GSK-3α), a coding sequence encoding the polypeptide of SEQ ID NO: 1, as shown in Fig. 10 (human GSK-3β), and Genbank L33801 (human GSK-3β). Preferably, the polynucleotide sequence encodes a human GSK polypeptide.

By a "high fat diet" is meant any diet commercially marketed for laboratory research as a high fat diet, or a diet in which at least 15% of calories come from fat, preferably, at least 40% of calories come from fat.

"Operably linked" refers to the situation in which a first nucleic acid sequence is placed in a functional relationship with a second nucleic acid sequence. For example, a regulatory sequence is operably linked to a coding sequence if the regulatory sequence functions to initiate/regulate transcription or expression of the coding sequence. Generally, operably linked DNA sequences may be in close proximity or contiguous and, where necessary to join two protein coding regions, in the same reading frame.

"Polynucleotide" generally refers to any RNA (e.g., mRNA), RNA-like, DNA (e.g., cDNA or genomic), or DNA like sequences, including, without limit, single-stranded, double-stranded, and triple-stranded sequence, sense or antisense strands, sequence generated using nucleotide analogs, hybrid molecules comprising RNA and DNA, and RNA or DNA containing modified bases. The polynucleotide can be naturally-occurring or synthesized.

"Transformation" or "transfection" describes a process of genetic modification by which heterologous DNA enters and renders a recipient cell capable of expressing the heterologous DNA. Transformation may occur in a prokaryotic or eukaryotic host cell according to various methods well known in the art. The method is selected based on the type of host cell being transformed and includes, but is not limited to, viral infection, electroporation, heat shock, lipofection, and particle bombardment. The terms "transformed cells" or "transfected cells" include stably transformed cells in which the inserted DNA is capable of replication either as an autonomously replicating plasmid or as part of the host chromosome, as well as transiently transformed or transfected cells which express the inserted DNA or RNA for limited periods of time. All of such transformed or transfected cells are referred to as "transgenic."

By a "transgenic non-human mammal" is meant a non-human mammal containing a heterologous transgene present as an extrachromosomal element or, preferably, present as a stably integrated element present in the chromosomal DNA of the mammal. Vectors for stable integration into the genome of a cell include plasmids, viruses, including retroviruses, and artificial chromosomes. The transgenic non-human mammal of the invention is originally produced, for example, by creating a blastocyst or embryo carrying the desired transgene and then implanting the blastocyst or embryo in a pseudopregnant foster mother for *in utero* development. A preferred method of making the transgenic blastocyst or embryo is by zygote injection in which DNA containing the desired transgene is injected into a fertilized egg or zygote. The transgenic blastocyst or embryo can also be made by first genetically-modifying an embryonic stem (ES) cell and then implanting the ES cell into a blastocyst or aggregating the ES cell with tetraploid embryos. Alternatively, various species of genetically-modified embryos can be obtained by nuclear transfer. In the case of nuclear transfer, the donor cell is a somatic cell or a pluripotent stem cell, and it is engineered to contain the desired transgene. The nucleus of this cell is then transferred into a fertilized or parthenogenetic oocyte that is enucleated, the embryo is reconstituted, and developed into a blastocyst. A genetically-modified blastocyst produced by any of the above methods is then implanted into a pseudopregnant foster mother according to standard methods well known to those skilled in the art. A "transgenic non-human mammal" includes all progeny of the mammal created by the methods described above, provided that the progeny inherit at least one copy of the transgene. It is preferred that all somatic cells and germline cells of the transgenic mammal contain the modification. Preferred transgenic non-human mammals of the invention include rodents, such as mice and rats, cats, dogs, rabbits, guinea pigs, hamsters, sheep, pigs, and ferrets.

By a "pseudopregnant" foster mother or female is meant a female in estrous who has mated with a vasectomized male; she is competent to receive embryos but does not contain any of her own endogenous fertilized eggs.

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art, and that are able to be ascertained without undue experimentation. Additional guidance with respect to making and using nucleic acids and polypeptides is found in standard textbooks of molecular biology, protein science, and immunology (see, e.g., Davis et al., *Basic Methods in Molecular Biology*, Elsevir Sciences Publishing, Inc., New York, NY,1986; Hames et al., *Nucleic Acid Hybridization*, IL Press, 1985; *Molecular Cloning,* Sambrook et al., *Current Protocols in Molecular Biology,* Eds. Ausubel et al., John Wiley and Sons; *Current Protocols in Human Genetics,* Eds. Dracopoli et al., John Wiley and Sons; *Current Protocols in Protein Science,* Eds. John E. Coligan et al., John Wiley and Sons; and *Current Protocols in Immunology,* Eds. John E. Coligan et al., John Wiley and Sons). All publications mentioned herein are incorporated by reference in their entireties.

### Description of the Figures

Fig. 1 is a Western blot that shows the presence of myc-tagged human GSK-3β expression in bigenic (+) mice skeletal muscle in mouse lines 1, 10, and 11 (but not 2), whereas no human GSK-3B expression was observed in the MLC2 control (-) lines. The myc-tagged human GSK-3β is visible in the bigenic lines as the 49 kd protein migrating between the endogenous murine GSK-3α (52 kd) and the endogenous murine GSK-3β (47 kd).
Fig. 2 is a Western blot that shows the inducible expression of myc-tagged human GSK-3β in muscle in doxycycline untreated (-) mice as compared to doxycycline treated (+) mice.
Fig. 3 is a bar graph showing the increase in muscle GSK-3β activity in bigenic lines 1, 10, and 11 (but not 2), as compared to the MLC2 transgenic control (CTL), consistent with the muscle tissue expression of human GSK-3B in these lines as shown in Fig. 1.
Fig. 4 is a bar graph showing the increase in plasma glucose in male bigenic lines 1, 10, and 11 as compared to the MLC2 transgenic controls.
Fig. 5 is a bar graph showing the increase in insulin levels in male bigenic lines 1, 10, and 11 as compared to the MLC2 transgenic controls.
Fig. 6 is a graph showing an increased weight gain in male bigenic lines on a high fat diet (HF) as compared to the transgenic MLC2 controls on a HF diet or on a chow diet.
Fig. 7 is a graph showing increased insulin levels in male bigenic lines on a HF diet as compared to the transgenic MLC2 controls on a HF diet or on a chow diet.
Fig. 8 is a graph showing increased glucose levels in male bigenic lines on a HF diet as compared to the transgenic MLC2 controls on a HF diet or on a chow diet.
Fig. 9A is a graph showing no significant change in liver glycogen content in male bigenic lines on a HF diet as compared to the transgenic MLC2 controls on a HF diet. Fig. 9B is a graph showing decreased muscle glycogen content in male bigenic lines on a HF diet as compared to the transgenic MLC2 controls on a HF diet.
Fig. 10 shows the polypeptide encoded by a human GSK-3β coding sequence (SEQ ID NO: 1).

### Detailed Description

### The GSK-3 Transgene Scheme

The transgenic non-human mammals of the invention contain a heterologous GSK-3 coding sequence operably linked to a promoter that drives expression in muscle in an inducible fashion. Preferably, the transgenic expression is muscle specific. In one embodiment, the promoter used to generate muscle specific expression is the myosin light chain (MLC) 2 promoter (e.g., the rat MLC2 sequence described in Nudel et al., Nucleic Acids Research 12: 7175-86, 1984; Marshall et al., J. Biol. Chem. 268: 18442-45, 1993; Genbank X00975). Another muscle specific promoter which can be used is the muscle creatine kinase promoter (Zabolotny et al., Proc. Natl. Acad. Sci. USA 98: 5187-92, 2001).

The GSK-3 coding sequence contained in the transgene is a vertebrate GSK-3 coding sequence, including either the GSK-3α or GSK-3β isoform. Typically, the transgene DNA is inserted into an expression vector (e.g., a plasmid or viral vector) and expressed in a yeast, mammalian, or bacterial expression system. For the generation of transgenic cells using microinjection, the transgene is first excised from the vector and then purified.

Expression of the GSK-3 transgene is inducible in the transgenic non-human mammals of the invention when certain conditions are present, according to a standard inducible expression scheme known in the art.

Inducible expression of the GSK-3 gene can be achieved by using a tetracycline responsive binary system (Gossen and Bujard, Proc. Natl. Acad. Sci. USA 89: 5547-51, 1992). This system involves genetically modifying a non-human to introduce two transgenes; one transgene encoding a tetracycline-inhibitable transcription factor (tTA) operably linked to a promoter that directs expression in muscle, and the other transgene encoding a GSK-3 coding sequence operably linked to tTA-responsive TetOp promoter. In these transgenic non-human mammals, the administration of tetracycline (or doxycycline) prevents expression of GSK-3. The transgene expression of GSK-3 is induced, therefore, by the termination of tetracycline administration to the animal (St.-Onge et al., Nucleic Acids Res. 24: 3875-77, 1996, U.S. Patent No. 5,922,927).

A recombinase excision system, such as a Cre-Lox system, may be used to activate GSK-3 gene expression under particular environmental conditions. Generally, methods utilizing Cre-Lox technology are carried out as described by Torres and Kuhn, *Laboratory Protocols for Conditional Gene Targeting,* Oxford University Press, 1997. Methodology similar to that described for the Cre-Lox system can also be employed utilizing the FLP-FRT system. Further guidance regarding the use of recombinase excision systems for inducible expression schemes is provided, for example, in U.S. Pat. No. 5,626,159, U.S. Pat. No. 5,527,695, U.S. Pat. No. 5,434,066, WO 98/29533, U.S. Pat. No. 6,228,639, Orban et al., Proc. Nat. Acad. Sci. USA 89: 6861-65, 1992; O'Gorman et al., Science 251: 1351-55, 1991; Sauer et al., Nucleic Acids Research 17: 147-61, 1989; Barinaga, Science 265: 26-28, 1994; and Akagi et al., Nucleic Acids Res. 25: 1766-73, 1997.

The transgenic, non-human mammals of the invention, wherein they contain two transgenes to express GSK-3 in muscle in an inducible fashion (bigenic), can be generated through standard transgenic techniques or by crossing transgenic non-human mammals wherein one parent contains one of the transgenes and the other parent contains the second transgene. Further guidance regarding the use of inducible schemes for GSK-3 transgene expression is found, for example, in Sauer, Meth. Enz. 225: 890-900, 1993, Gu et al., Science 265: 103-06, 1994, Araki et al., J. Biochem. 122: 977-82, 1997, Dymecki, Proc. Natl. Acad. Sci. 93: 6191-96, 1996, and Meyers et al., Nature Genetics 18: 136-41, 1998.

### Transgenic Non-human Mammals and Animal Cells

There are a number of techniques which permit the introduction of the GSK-3 transgene into an embryo that is then developed into a transgenic non-human mammal of the invention. The most commonly used protocol involves the direct injection of the GSK-3 transgene into the pronucleus of a fertilized egg, preferably a male fertilized egg (Hogan et al., *Manipulating the Mouse Embryo (A Laboratory Manual)*, Cold Spring Harbor Laboratory Press, Cold Spring Harbor, NY, 1994, 2^{nd} edition, Breban et al., J. Immunol. 156: 794, 1996; Gariepy et al., J. Clin. Invest. 102: 1092, 1998), or into a cell of a zygote. The genetically-modified embryos that develop by this process may have one or several copies of the transgene integrated into their genomes, usually in one integration site.

An alternative method to introduce the transgene into a non-human mammalian embryo used to make the transgenic non-human mammals of the invention involves transfecting ES cells such that they contain the GSK-3 transgene according to methods known in the art, such as electroporation, infection with retroviral vectors, or lipofection, and then implanting the ES cells into suitable blastocyst hosts (Capecchi, Trends Genet. 5: 70; 1989) or associating the ES cell with a tetraploid embryo. This technique is especially successful for transfection with very large human artificial chromosomes (HACs), bacterial artificial chromosomes (BACs), or yeast artificial chromosomes (YACs) (Hodgson et al., Neuron 23: 181, 1999; Lamb et al., Nature Neuroscience 2: 695, 1999). Due to positional effects, expression of a randomly integrated transgene may be inhibited or occur in a non-authentic manner with respect to the chosen promoter. To overcome these potential problems, transgenes can be inserted into predetermined loci (e.g., ROSA26 or HPRT) that support transcriptional activity without adverse effects (Zambrowicz et al., Proc. Natl. Acad. Sci. USA 94: 3789, 1997; Soriano et al., Nature Genetics 21: 70, 1999).

Regardless of how the genetically modified embryos or blastocysts are created, they are then implanted in pseudopregnant female mice and allowed to develop *in utero* (Hogan et al., 1994, *supra*; *Teratocarcinomas and Embryonic Stem Cells: A Practical Approach,* E.J. Robertson, ed., IRL Press, Washington, D.C., 1987).

The transgenic offspring born to the pseudopregnant females are termed "founder" animals. The animals are tested to ensure the desired genotypic change has occurred. This can be done, for example, by detecting the presence of the transgene by PCR with specific primers, or by Southern blotting of tail DNA with a transgene-specific probe (Erlich et al., Science 252: 1643-51, 1991; Zimmer and Gruss, Nature 338: 150, 1989; Mouellic et al., Proc. Natl. Acad. Sci. (USA) 87: 4712, 1990; and Shesely et al., Proc. Natl. Acad. Sci. (USA) 88: 4294, 1991).

Once the desired genotype has been confirmed, the transgenic non-human mammals are bred to establish transgenic lines and then backcrossed into the genetic background of choice. It is convenient to have the transgene insertion on both chromosomes as this eliminates the need for repeated genotyping in the course of routine animal husbandry.

The transgenic line is subjected to various tests to determine whether the desired gain-of-function phenotype is present. The transgenic non-human mammals of the invention have a sufficient level of expression of the GSK-3 transgene to result in hyperinsulinemia, hyperglycemia, increased weight gain resulting from a high fat diet, and/or decreased muscle glycogen content. These phenotypes can be determined by any method known in the art, including the methods described in the Examples herein.

Muscle cells, e.g., myoblasts or myocytes, of the invention are isolated from the above-described non-human mammals and are used in GSK-3 screening assays. The transgenic muscle cells of the invention demonstrate abnormal insulin-stimulated glucose uptake, protein synthesis, apoptosis, and/or differentiation.

### Screening Assays

The above-described phenotypes of the transgenic non-human mammals and animal cells of the invention demonstrate that the transgenic non-human mammals and animal cells are useful as models of insulin resistance, diabetes, and/or obesity. Thus, these transgenic non-human mammals and animal cells are useful tools in screening assays to identify agents that modulate GSK-3 activity, preferably, agents that decrease GSK-3 activity. These agents that decrease GSK-3 activity can be useful as therapeutics for treating metabolic disorders associated with insulin resistance, diabetes, and/or obesity.

Agents that modulate GSK-3 activity can be identified by administering the agent to the transgenic non-human mammals of the invention, in which transgenic expression has been induced, and assessing GSK-3 activity by assessing insulin levels, body weight gain associated with a high fat diet, or an increase in muscle glycogen content. Agents that decrease GSK-3 activity in the transgenic non-human mammals of the invention will also decrease insulin, decrease glucose, decrease the body weight gain associated with a high fat diet, and/or increase muscle glycogen content associated with a high fat diet, as compared to the transgenic non-human mammals of the invention in the absence of the agent. Based upon the present invention, one skilled in the art will recognize that standard methods for measuring GSK-3 activity (Nikoulina et al., *supra;* Cross et al., FEBS Lett. 406: 211-215, 1997; Hurel et al., Biochem. J. 320: 871-77, 1996; Biochem. J. 303: 21-26, 1994; Biochem. Biophys. Res. Commun 210: 738-45, 1995), insulin, body weight, and muscle glycogen content can be used to perform the assay methods using the transgenic non-human mammals of the invention. Exemplary methods are also provided in the Examples herein.

The test agents used for screening may first be identified as GSK-3 modulators by *in vitro* or cell-based assay, or selected randomly. Test agents may be selected individually or obtained from a compound library. Such libraries include biological libraries, peptoid libraries (libraries of molecules having the functions of peptides, but with novel, non-peptide backbones which are resistant to enzymatic degradation yet remain bioactive) (see, e.g., Zuckermann, J. Med. Chem. 37:2678-85, 1994), spatially addressable parallel solid phase or solution phase libraries, synthetic library methods requiring deconvolution, the "one-bead one-compound" library method, and synthetic library methods using affinity chromatography selection.

Examples of methods for the synthesis of molecular libraries can be found in the art, for example, in: DeWitt et al., Proc. Natl. Acad. Sci. (USA) 90:6909 (1993); Erd et al., Proc. Natl. Acad. Sci. (USA) 91: 11422, 1994; Zuckermann et al., J. Med. Chem., 37:2678,1994; Cho et al., Science, 261:1303, 1995; Carrell et al., Angew. Chem. Int. Ed. Engl. 33:2061, 1994; and in Gallop et al., J. Med. Chem. 37:1233, 1994.

Compounds may be presented to the transgenic non-human mammals in solution or in suspension via any appropriate route of administration, e.g., orally, intraperitoneally, intramuscularly, intravenously, or intraventricularly. In cell based assays, compounds or libraries of compounds may be presented in solution (e.g., Houghten, Biotechniques, 13: 412-421, 1992), or on beads (Lam, Nature 354: 82-84,1991), on chips (Fodor, Nature 364: 555-556, 1993), bacteria or spores (Ladner, U.S. Patent No. 5,223,409), plasmids (Cull et al., Proc. Natl. Acad. Sci. USA. 89: 1865-1869, 1992) or on phage (Scott et al., Science 249: 386-390, 1990; Devlin, Science 249: 404-406, 1990; Cwirla et al., Proc. Natl. Acad. Sci. (USA) 87: 6378-6382, 1990; Felici, J. Mol. Biol. 222: 301-310, 1991; Ladner, *supra).*

### Example 1 - Creation of the Bigenic Mice

The bigenic mice were designed to contain two transgenes: one transgene encoding a tetracycline-inhibitable transcription factor (tTA) under the control of a rat myosin light chain (MLC) promoter (MLC-tTA), and the other transgene encoding a myc-tagged human GSK-3 sequence under the control of the tTA-responsive TetOp promoter (pTetOp-GSK-3B) (Gossen and Bujard, Proc. Natl. Acad. Sci. USA 89: 5547-51, 1992; Science 268: 1766-69, 1995). The bigenic mice were created by crossing MLC-tTA transgenic mice with pTetOp-GSK-3B transgenic mice.

For the MLC-tTA plasmid, pHelix (Roche Diagnostics Corp., Indianapolis, IN) was used as the cassette backbone. The plasmid contained a 1.2 Kb fragment of the rat myosin light chain 2 promoter (Nudel et al., Nucleic Acids Research 12: 7175-86, 1984; Marshall et al., J. Biol. Chem. 268: 18442-45, 1993; Genbank X00975), the tTA sequence (Gossen and Bujard, 1992 and 1995, *supra),* and SV40 polyadenylation signal. A Nhel/Notl digest of the tTA-polyA sequence was cloned into the SpeI/NotI site of the pBluescriptSK-(Stratagene, La Jolla, CA). The Nhel/Notl ends of the 1.2 Kb myosin light chain promoter fragment were blunt ended with T4 polymerase and cloned into the EcoRV site of the pBluescriptSK-. The MLC2-tTA-polyA transgene was then cut out of the pBluescriptSK- by XhoI/SwaI digest, blunt ended, and then cloned into the pHelix BamHl site. The transgene was isolated by Swa I digest.

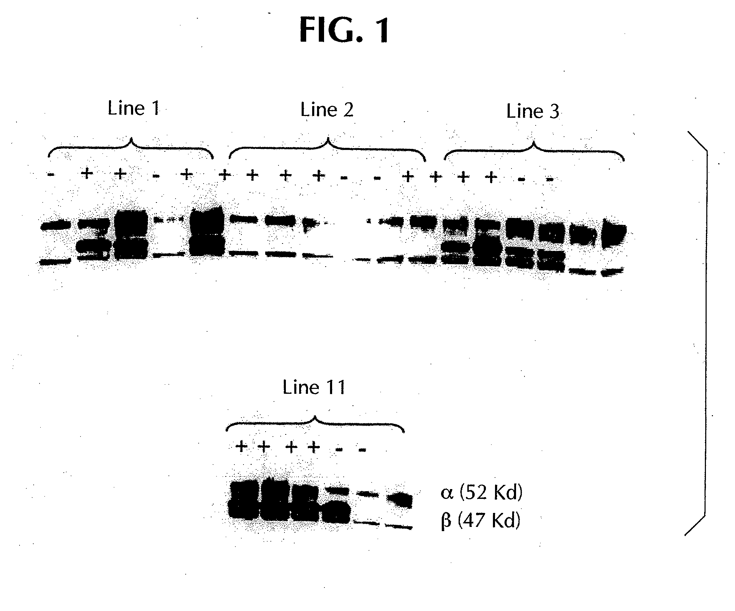

For the pTetOp transgene-GSK-3β plasmid, the TetOp promoter was obtained from a pBluescriptll KS+ TetOp CMV/HGH polyA vector. The GSK-3β coding sequence, encoding the polypeptide as shown in Fig. 1 (SEQ ID NO: 1), was blunt end ligated into the linearized TetOp CMV/HGH polyA vector. The functional transgene was cut out with BssHll digestion, purified by gel extraction, treated with T4 DNA and Qiagen PCR purification, and ligated into a linearized pHelix(1)- vector with double chromatin insulators (Chung et al., Proc. Natl. Acad. Sci. USA 94: 575-80, 1997) using T4 DNA ligase.

Each transgene was purified, gel isolated, and microinjected into separate one day old FVB/N mouse embryos (Taconic Labs, Germantown, NY). The embryos were implanted in pseudopregnant foster mothers.

All transgenic and bigenic mice, aged 0-4 weeks, and their (foster) mothers, were supplied with 100 µg/ml doxcycline (Sigma Chemicals, St. Louis, MO, #D-9891) to prevent transcription of the GSK-3β transgene. The water was stored in colored plastic bottles to protect against doxycycline degradation by the light. Water bottles for the mice were changed weekly. After 4 weeks of age in the bigenic mice and in the MLC2 transgenic controls, doxycycline was removed from the water to allow human GSK-3β transcription to proceed in muscle.

At five months of age, bigenic and MLC2 transgenic control mice were sacrificed and skeletal muscle (0.4 grams) was homogenized in 10 volumes of homogenization buffer (50 mM TRIS pH 8.0, 10 mM β-glycerophosphate, 5 mM EGTA, 50 mM NaCI, 10 mM DTT, 1 µM microcystin, 1 mM NaVO₄, 1 mM benzamidine (all from Sigma Chemical, St. Louis, MO), 1x Protease Inhibitor Cocktail (Calbiochem, San Diego, CA)) by polytron for two 30-second bursts on ice. Homogenates were centrifuged at 17,000xg for 10 min. at 4°C. Supernatants were recovered and protein determined by Bradford assay (Bio-Rad Laboratories, Hercules, CA).

Skeletal muscle homogenates were prepared and analyzed for GSK-3β expression by immunoblotting. Homogenates (15µg) were resolved on reducing/denaturing (SDS) 4-12% gels (Novex, Carlsbad, CA) and transferred to nitrocellulose. Membranes were blocked and immunoblotted in 3% non-fat milk in phosphate buffered saline containing 0.1% Tween-20 (Sigma Chemical). Human GSK-3β protein was detected using a monoclonal antibody (UBI, Lake Placid, NY, #05-412) at 1 µg/ml for 90 minutes. Blots were stripped and immunoblotted with a polyclonal anti-phospho-GSK-3β (S21/9) antibody (Cell Signaling, Beverly, MA, #9331S) at 1:1000 dilution overnight at 4°C. Blots were stripped again and tyrosine phosphorylated GSK-3β was detected using an anti-phospho-GSK-3β (Y279/216) monoclonal antibody (UBI, #05-413) at 1 µg/ml for 2 hours at room temperature. Detection was accomplished using appropriate secondary antibodies conjugated to horseradish peroxidase (Sigma) followed by enhanced chemiluminescence using SuperSignal® West Pico (Pierce, Rockford, IL).

Fig. 1 shows the presence of human GSK-3β expression in bigenic (+) mice skeletal muscle in mouse lines 1,10, and 11, whereas no human GSK-3B expression was observed in the MLC2 control (-) lines. The human GSK-3β is visible in the bigenic lines as the 49 kd protein migrating between the endogenous murine GSK-3α (52 kd) and the endogenous murine GSK-3β (47 kd).

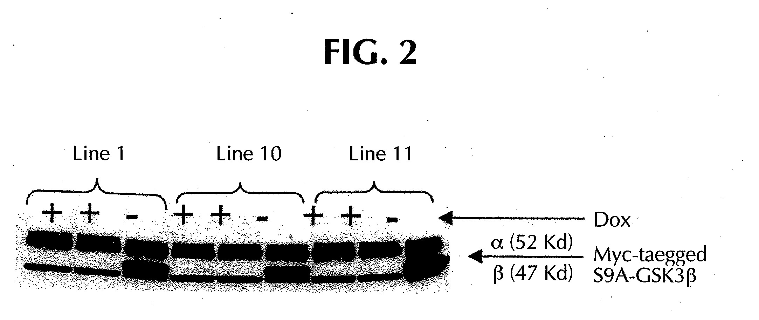

At five months of age, doxycycline treatment (100 µg/ml) was resumed in the drinking water of another set of bigenic mice from lines 1, 10, and 11 to confirm that the GSK-3 expression was indeed inducible and not constitutive. Fig. 2 shows that the resumption of doxycycline was sufficient to eliminate transgenic expression of the human GSK-3β.

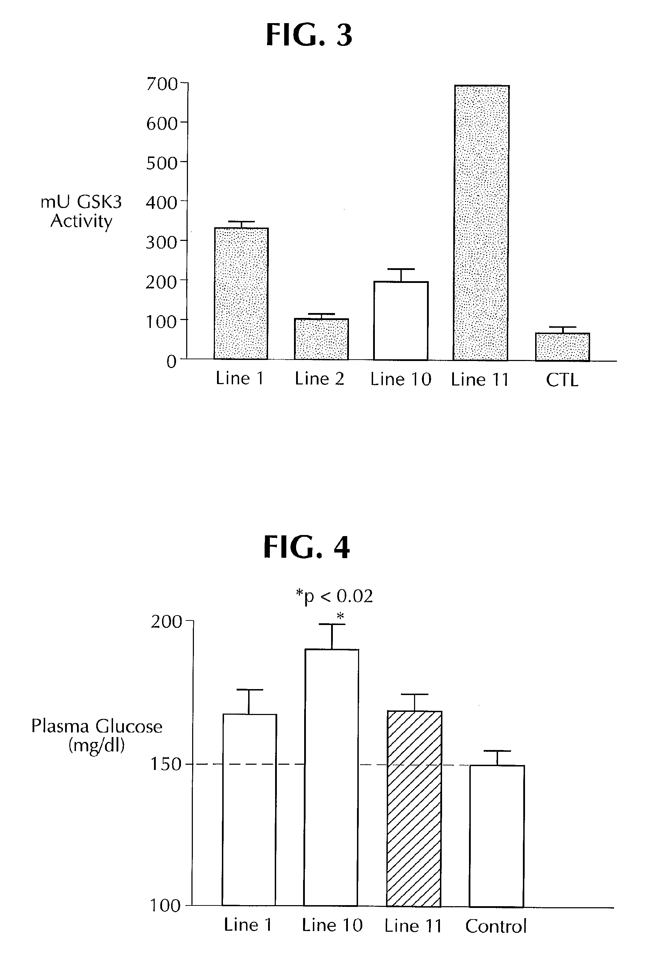

Skeletal muscle homogenates from five month old bigenic and MLC2 transgenic control mice (in which doxycycline treatment was terminated at four weeks of age) were also prepared for analysis of GSK-3β activity. Muscle homogenates (6µg) in 25µl of homogenization buffer were mixed with 12.5µl of 1.11 mM substrate peptide (B-2B-pS (Anaspec, Inc., San Jose, CA) based on the primed eIF2B consensus sequence for GSK-3 phosphorylation: biotin-RRAAEELDSRAGpSPQL-OH (SEQ ID NO: 2)), and the reaction was started upon addition of 12.5 µl 4x ATP mix (200 mM TRIS-HCI pH 7.4, 50 mM MgCl₂, 8 mM DTT, 0.4 mM ATP and 0.08 µCi/µl ³³P-gamma-ATP (3000 Ci/mmol) (Perkin Elmer Life Sciences, Boston, MA, #NEG602H). Incubations were carried out at 30°C; 9 µl aliquots of reaction mix were removed at 5, 10, 20, and 30 min. and the reaction was stopped by mixing with 9 µl of 1 N HCI on ice. Stopped reaction mix (10 µl) was spotted onto streptavidin-coated paper squares (SAM2 membranes, Promega, Madison, WI). Squares were washed 1x 30 sec. and 3x 2 min. in 2 M NaCI, followed by 4x 2 min. in 2 M NaCl/1% phosphoric acid, and then 2x in distilled water and once in ethanol. The squares were dried and counted on a Wallac scintillation counter (Perkin Elmer Life Sciences, Boston, MA). The dpm's were converted to pmoles product formed and graphed as a function of time. The slopes were determined by linear fit using GraphPad Prism® (GraphPad Software Inc., San Diego, CA) and are equivalent to mU GSK-3β activity (pmoles product formed per minute). As shown in Fig. 3, muscle GSK-3β activity was increased over control in lines 1, 10, and 11, consistent with the muscle tissue expression of human GSK-3B in these lines as shown in Fig. 1.

### Example 2 - Phenotypic Characterization of MLC2-GSK-3β Bigenic Mice

### 1. Plasma Glucose

Non-anaesthetized, *ad libitum* fed five month old male bigenic and MLC2 transgenic mice were bled (25 µl) via orbital sinus with a micropipette. The blood was immediately diluted into 100 µl of 0.025% heparin in normal saline. Red cells were pelleted by centrifugation and plasma glucose levels were determined with a Roche/Hitachi 912 Analyzer (Boehringer Mannheim, Indianapolis, IN). Raw data was multiplied by a dilution factor of 8.14 to account for hematocrit and plasma dilution. The bigenic mice demonstrated elevated glucose levels (Fig. 4).

### 2. Plasma Insulin

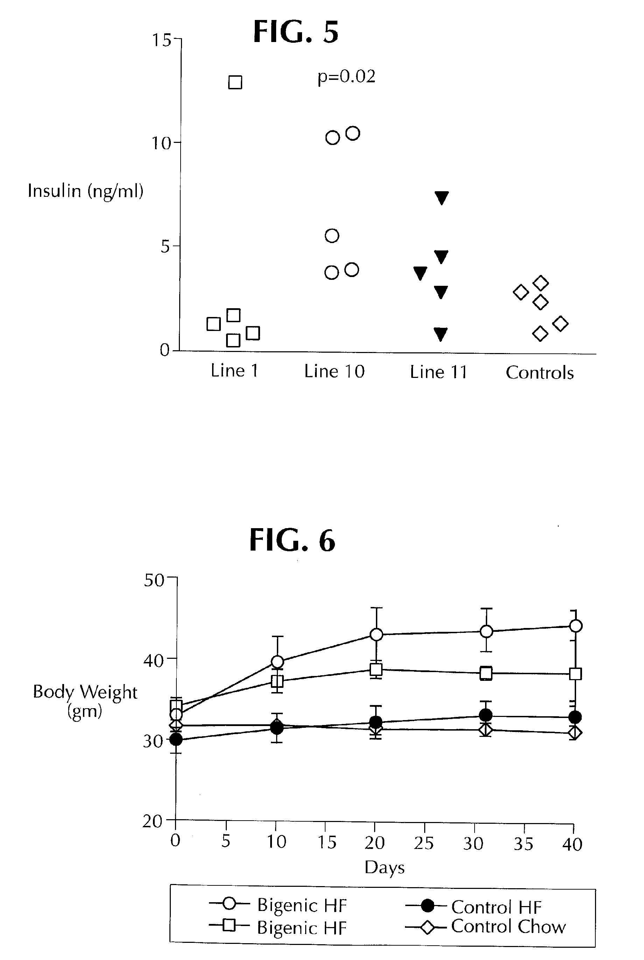

At five months of age, *ad libitum* fed male mice were sacrificed and blood collected into Microtainer tubes containing lithium heparin (Becton Dickenson, Franklin Lakes, NJ). The blood was centrifuged and plasma collected. Insulin levels were measured in 25µl aliquots by ELISA (Alpco, Windham, NH). Fig. 5 demonstrates that plasma insulin levels were elevated in the bigenic mice as compared to MLC2 transgenic controls.

### 3. Effects of a High Fat Diet on Body Weight, Insulin Levels, Glucose Levels, and Glycogen Content

Bigenic and MLC2 male transgenic mice ranging from 5-7 months of age were switched from a control diet (Research Diets, Inc., New Brunswick, NJ, #5001) to a high fat diet (Research Diets, Inc., #D12331) for 40 days. Body weights were measured throughout the study. At the termination of the study, blood glucose and insulin levels were determined as previously described. Muscle and liver glycogen levels were also determined in tissue digests according to the methods described in Hassid and Abraham's Methods in Enzymology 3: 34-35, 1959.

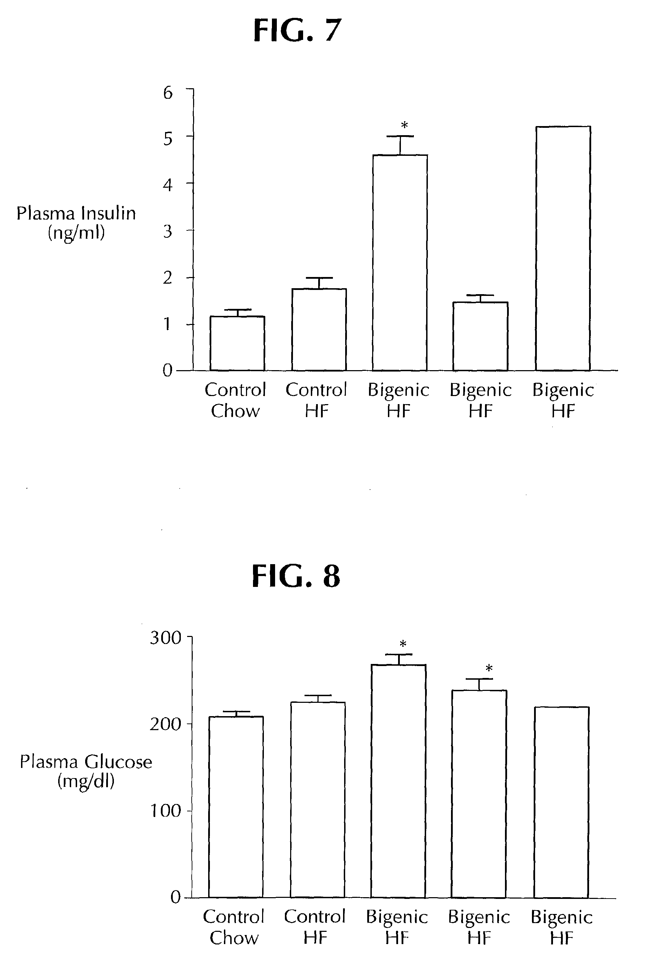

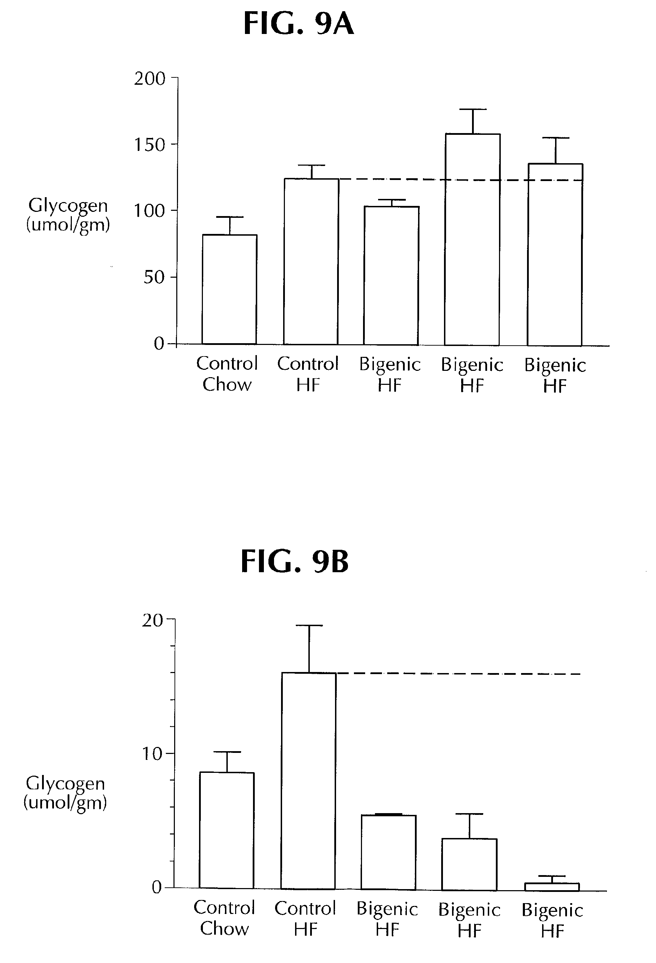

As shown in Fig. 6, the high fat diet caused a much greater weight gain in the GSK-3 bigenic lines than in the MLC transgenic control line. Plasma insulin levels were also dramatically increased in two of the three GSK-3 bigenic lines as compared to the MLC2 transgenic control following the high fat diet regime (Fig. 7), and plasma glucose levels were elevated in the GSK-3 bigenic lines (Fig. 8). As shown in Fig. 9B, GSK-3 transgenic expression in muscle reduced the muscle glycogen content in the GSK-3 bigenic mice as compared to the transgenic MLC2 controls following the high fat diet. This effect was not evident in the liver, however, where glycogen contents were similar in the GSK-3 bigenic mice and the MLC2 transgenic controls (Fig. 9A).

## Claims

1. A transgenic non-human mammal expressing or capable of expressing a GSK-3 transgene comprising a GSK-3 coding sequence, wherein expression of said coding sequence in said mammal is driven by an operably linked regulatory sequence that directs inducible expression in muscle, and wherein, following induction of transgene expression, said mammal exhibits hyperinsulinemia, hyperglycemia, increased weight gain on a high fat diet, and/or decreased muscle glycogen content on a high fat diet, as compared to a control mammal not expressing the GSK-3 transgene.

2. The transgenic non-human mammal of claim 1, wherein said inducible expression is muscle specific.

3. The transgenic non-human mammal of claim 1, wherein said regulatory sequence comprises a myosin light chain promoter.

4. The transgenic non-human mammal of claim 1, wherein said mammal is a rodent.

5. The transgenic non-human mammal of claim 4, wherein said rodent is a mouse.

6. The transgenic non-human mammal of claim 1, wherein said GSK-3 transgene is a human GSK-3α or GSK-3β coding sequence.

7. The transgenic non-human mammal of claim 1, wherein said GSK-3 transgene comprises the human GSK-3β coding sequence encoding the polypeptide of SEQ ID NO: 1.

8. A method of identifying an agent that modulates GSK-3 activity *in vivo*, said method comprising contacting said agent with a transgenic non-human mammal expressing a transgene comprising a GSK-3 coding sequence, wherein expression of said coding sequence in said mammal is driven by an operably linked regulatory sequence that directs inducible expression in muscle, and wherein said non-human mammal exhibits hyperinsulinemia, hyperglycemia, increased weight gain on a high fat diet, and/or decreased muscle glycogen content on a high fat diet, as compared to a control animal not expressing the GSK-3 transgene, wherein a difference in insulin, glucose, body weight on a high fat diet, and/or muscle glycogen content on a high fat diet, in the presence of the agent and in the absence of the agent, is indicative that the agent modulates said activity.

9. A muscle cell or myoblast isolated from the transgenic non-human mammal of claim 1.

10. The muscle cell or myoblast of claim 9, wherein said muscle cell or myoblast is immortalized.


SEQUENCE LISTING

<110>  Pfizer Products Inc.

<120>  TRANSGENIC EXPRESSION OF GLYCOGEN SYNTHASE KINASE 3 IN MUSCLE

<130>  PC23139AUS

<150>  60/343,479
<151>  2002-12-21

<160>  2

<170>  PatentIn version 3.1

<210>  1
<211>  420
<212>  PRT
<213>  Homo sapiens

<400>  1

Met Ser Gly Arg Pro Arg Thr Thr Ala Phe Ala Glu Ser Cys Lys Pro
1               5                   10                  15

Val Gln Gln Pro Ser Ala Phe Gly Ser Met Lys Val Ser Arg Asp Lys
            20                  25                  30

Asp Gly Ser Lys Val Thr Thr Val Val Ala Thr Pro Gly Gln Gly Pro
        35                  40                  45

Asp Arg Pro Gln Glu Val Ser Tyr Thr Asp Thr Lys Val Ile Gly Asn
    50                  55                  60

Gly Ser Phe Gly Val Val Tyr Gln Ala Lys Leu Cys Asp Ser Gly Glu
65                  70                  75                  80

Leu Val Ala Ile Lys Lys Val Leu Gln Asp Lys Arg Phe Lys Asn Arg
                85                  90                  95

Glu Leu Gln Ile Met Arg Lys Leu Asp His Cys Asn Ile Val Arg Leu
            100                 105                 110

Page 1

Arg Tyr Phe Phe Tyr Ser Ser Gly Glu Lys Lys Asp Glu Val Tyr Leu
115 120 125

Asn Leu Val Leu Asp Tyr Val Pro Glu Thr Val Tyr Arg Val Ala Arg
130 135 140

His Tyr Ser Arg Ala Lys Gln Thr Leu Pro Val Ile Tyr Val Lys Leu
145 150 155 160

Tyr Met Tyr Gln Leu Phe Arg Ser Leu Ala Tyr Ile His Ser Phe Gly
165 170 175

Ile Cys His Arg Asp Ile Lys Pro Gln Asn Leu Leu Leu Asp Pro Asp
180 185 190

Thr Ala Val Leu Lys Leu Cys Asp Phe Gly Ser Ala Lys Gln Leu Val
195 200 205

Arg Gly Glu Pro Asn Val Ser Tyr Ile Cys Ser Arg Tyr Tyr Arg Ala
210 215 220

Pro Glu Leu Ile Phe Gly Ala Thr Asp Tyr Thr Ser Ser Ile Asp Val
225 230 235 240

Trp Ser Ala Gly Cys Val Leu Ala Glu Leu Leu Leu Gly Gln Pro Ile
245 250 255

Phe Pro Gly Asp Ser Gly Val Asp Gln Leu Val Glu Ile Ile Lys Val
260 265 270

Leu Gly Thr Pro Thr Arg Glu Gln Ile Arg Glu Met Asn Pro Asn Tyr
275 280 285

Thr Glu Phe Lys Phe Pro Gln Ile Lys Ala His Pro Trp Thr Lys Val
290 295 300

Phe Arg Pro Arg Thr Pro Pro Glu Ala Ile Ala Leu Cys Ser Arg Leu
305 310 315 320

Leu Glu Tyr Thr Pro Thr Ala Arg Leu Thr Pro Leu Glu Ala Cys Ala
325 330 335

His Ser Phe Phe Asp Glu Leu Arg Asp Pro Asn Val Lys Leu Pro Asn
340 345 350

Gly Arg Asp Thr Pro Ala Leu Phe Asn Phe Thr Thr Gln Glu Leu Ser
355 360 365

Ser Asn Pro Pro Leu Ala Thr Ile Leu Ile Pro Pro His Ala Arg Ile
370 375 380
Page 2

Gln Ala Ala Ala Ser Thr Pro Thr Asn Ala Thr Ala Ala Ser Asp Ala
385                 390                 395                 400

Asn Thr Gly Asp Arg Gly Gln Thr Asn Asn Ala Ala Ser Ala Ser Ala
                405                 410                 415

Ser Asn Ser Thr
            420

<210>  2

<211>  17

<212>  PRT

<213>  synthetic

<400>  2

Arg Arg Ala Ala Glu Glu Leu Asp Ser Arg Ala Gly Pro Ser Pro Gln
1               5                   10                  15

Leu